# EUROPÄISCHE PATENTSCHRIFT

(11) **EP 2 533 752 B1**
(45) Veröffentlichungstag und Bekanntmachung des Hinweises auf die Patenterteilung: **03.03.2021**
(21) Anmeldenummer: 11702791.2
(22) Anmeldetag: 26.01.2011
(51) Int. Cl.: A61K 8/19, A61Q 19/00, A61Q 7/00, A61K 8/73, A61K 8/67, A61K 8/60, A61K 8/55, A61K 8/49, A61K 8/41, A61Q 19/08, A61K 8/02, A61K 8/9711, A61K 8/9761, A61K 8/9771, A61K 8/9794, A61K 8/9789

(54) **ZUBEREITUNGEN ZUR STABILISIERUNG / AUFBAU DER TEXTUR LEBENDER GEWEBE WIE HAUT, UND DEREN ANWENDUNGEN**
PREPARATIONS FOR STABILISING/FORMING THE TEXTURE OF LIVING TISSUE SUCH AS SKIN, AND USES THEREOF
PRÉPARATIONS POUR STABILISER/ÉLABORER LA TEXTURE D'UN TISSU VIVANT COMME LA PEAU, ET LEURS APPLICATIONS

(30) Priorität: 12.02.2010 DE 102010007736
(43) Veröffentlichungstag der Anmeldung: 19.12.2012
(73) Patentinhaber: Börlind Gesellschaft für kosmetische Erzeugnisse mbH, 75365 Calw (DE)
(72) Erfinder: BEUTLER, Ralf., 64739 Höchst/Hummetroth (DE); SCHMIDT, Karlheinz., 72819 Gomaringen (DE)
(74) Vertreter: Müller, Claudia
(86) Internationale Anmeldenummer: PCT/EP2011/000340
(87) Internationale Veröffentlichungsnummer: WO 2011/098216

(56) Entgegenhaltungen:
- EP-A1- 1 609 461
- WO-A2-02/076423
- FR-A1- 2 573 651
- US-A1- 2006 198 810
- DATABASE GNPD [Online] MINTEL; December 2009 (2009-12), anonymous: "Rejuvenating Eye Cream", Database accession no. 1239969
- DATABASE GNPD [Online] MINTEL; November 2009 (2009-11), anonymous: "Facial Moisturising Mask", Database accession no. 1207851
- DATABASE GNPD [Online] MINTEL; October 2009 (2009-10), anonymous: "Shampoo", Database accession no. 1190378
- DATABASE GNPD [Online] MINTEL; September 2009 (2009-09), anonymous: "Night cream with cranberry juice", Database accession no. 1173933
- DATABASE GNPD [Online] MINTEL; June 2009 (2009-06), anonymous: "C is Smooth Body Moisturising Lotion", Database accession no. 1110778
- DATABASE GNPD [Online] MINTEL; 6 June 2008 (2008-06-06), anonymous: "Healthy Skin with Greens+ Drink Powder", XP055668868, retrieved from www.gnpd.com Database accession no. 919500
- DATABASE GNPD [Online] MINTEL; 19 September 2008 (2008-09-19), anonymous: "Liquid Dietary Supplement", XP055668861, retrieved from www.gnpd.com Database accession no. 975872
- DATABASE GNPD [Online] MINTEL; 2 April 2009 (2009-04-02), anonymous: "Dietary Supplement", XP055668873, retrieved from www.gnpd.com Database accession no. 1081422
- DATABASE GNPD [Online] MINTEL; 22 September 2003 (2003-09-22), anonymous: "Women's Blend Multi-Vitamin Supplement", XP055668870, retrieved from www.gnpd.com Database accession no. 10148652
- Anonymous: "Soybean - Wikipedia, the free encyclopedia", , 1 March 2015 (2015-03-01), pages 1-21, XP055174943, Retrieved from the Internet: URL:http://en.wikipedia.org/wiki/Soybean [retrieved on 2015-03-09]

## Beschreibung

### Gegenstand der Erfindung

Die vorliegende Erfindung betrifft insbesondere synergistisch wirkende Zubereitungen zur Stabilisierung und zum Aufbau (Forming) der Textur lebender Gewebe, insbesondere menschlicher Haut und ihrer Anhangsgebilde wie z.B. Haarfollikel und Nagelbett sowie Bindegewebe. Stabilisierung kann insofern erfolgen durch Texturaufbau und Texturneubildung, insbesondere der Haut. Die Zubereitungen umfassen eine Kombination aus 4 Grundbestandteilen A+B +C und D, nämlich: A) Inhibitoren des Abbaus der Gewebematrix durch Collagenase, Elastase und Hyaluronidase, B) Stimulatoren des Gewebematrix-Aufbaus, C) Aktivatoren der Blutgefäß-Funktion sowie D) Aktivatoren der Nervenfunktion (neurotrope Substanzen). Überraschenderweise kann mit einer derartigen Kombination A+B, +C +D, in synergistischer Weise degenerativen Prozessen im Sinne des Abbaus der Gewebetextur entgegengewirkt werden.

Dabei werden die Substanzen A) ausgewählt aus A1) Terpenoiden, wie vor allem Sesqui- und Triterpenhaltigen Pflanzenextrakten; oder aus A2) Phytoaciden Komponenten wie Phytosäuren, oder Fettsäurehaltigen (phytoiden) Pflanzenstoffen;; welche Inhibitoren der Collagenase, der Elastase und / oder Hyaluronidase darstellen, wie nachfolgend erläutert. Die Substanzen B) werden ausgewählt aus säurehaltigen Substanzen wie Blaubeeren-/ Heidelbeeren- / Preiselbeerenextrakt, Hibiscus-Extrakt, Morinda citrifolia (Nonifrucht), Granatapfelschalenextrakt, Paprikapulver. Substanzen der Gruppe C) werden gewählt aus gefäßaktiven Substanzen wie Calendula, Tagetesblüten- Weiße- Lilien- Extrakt, Tomatenextrakt, Niacin; Substanzen der Gruppe D) sind ausgewählt aus B- Vitaminen, ausgewählt aus B1, B2, B5, B6, B12, Getreideauszügen wie Buchweizenextrakt.

Die Wirkstoffe A, B, C, D der Zubereitungen liegen kombiniert vor, sowohl in topischer als auch in systemischer Verabreichungsform, die galenischen Mittel zur systemischen Verabreichung in Form von Dragees, Kapseln oder Tabletten und zur topischen Verabreichung in Form von Emulsionen/Lotionen, Cremes, Salben, Gelen, Fluids, Sprays vorliegen.

Die erfindungsgemäßen Zubereitungen können zusammen mit für die gewünschte Verabreichung geeigneten Zusatzstoffen einschl. Zusatzwirkstoffen kombiniert werden. Hierzu gehören insofern bekannte galenische Mittel zur systemischen Verabreichung in Form von Dragees, Kapseln, oder Tabletten. Eine e topische Verabreichung ist in Form von Emulsionen / Lotionen, Cremes, Salben, Gelen (z. B. wässrig/ ölig), Fluids, Sprays möglich.

### Stand der Technik

Der Erhalt von Struktur und Funktion der Textur lebender Gewebe basiert auf einem Gleichgewicht zwischen Reaktionen, die dem Aufbau und Abbau von Gewebebestandteilen dienen. Störungen dieses Gleichgewichts gehen mit veränderten anabolen oder katabolen Prozessen einher, wie dies beispielsweise bei vielen Belastungen oder Erkrankungen sowie bei der Alterung bekannt ist. Insbesondere verstärkte abbauende, katabole Prozesse können zu schwerwiegenden degenerativen Konsequenzen bis hin zur Zerstörung der Gewebetextur führen. Ebenso können unzureichende Aufbauprozesse zu einer negativen Bilanz mit entsprechenden strukturellen und funktionellen Gewebedefiziten führen.

Eine wesentliche Begleiterscheinung der strukturellen Alterung lebender Gewebe ist der Verlust an texturbildenden Proteinen und Proteoglykanen, die neben Wasser den größten Anteil der extrazellulären Matrix darstellen und zum Aufbau und zur Stabilisierung der Gewebetextur beitragen. Wichtige Bausteine hierfür sind dabei insbesondere Collagen, Elastin und Hyaluronsäure. Vor allem bei altersbedingten degenerativen Prozessen an Stütz- und Bindegewebe lassen sich die Gewebetexturveränderungen der Haut und ihrer Anhangsgebilde feststellen. Ausgangspunkt für verstärkte Abbauprozesse ist dabei die Aktivierung hydrolytischer Enzyme wie Collagenase, Elastase, Hyaluronidase.

Daneben können auch eingeschränkte Aufbauprozesse zu einer Instabilität führen, wie z. B. eine unzureichende Stimulation biosynthetischer Prozesse, eine unzureichende nutritive Versorgung z. B. mit Energie, eine unzureichende Gefäßversorgung oder eine mangelnde Reizsetzung. Insbesondere spielen auch antioxidative Prozesse eine wesentliche Rolle bei derartigen Prozessen.

Entsprechend dieser verschiedenen möglichen Mechanismen werden zur Beeinflussung der Textur lebender Gewebe wie Haut, Bindegewebe unterschiedliche Wege beschritten. Einerseits kann durch externe Applikation eine Einwirkung erfolgen, wie z. B. bei koffeinhaltigen Produkten. Auch sind Pflanzenextrakt-haltige Mittel bekannt, wie z. B. solche, enthaltend Granatapfelextrakt als antioxidativem Wirkstoff (WO 00/ 64472 A1). In der EP 1239 813 B1 wird die Verwendung eines Magnesium- angereicherten Blaualgenextraktes vorgeschlagen. Auch hier soll insbesondere die Wirkung antioxidativer Inhaltsstoffe (Katalase, SOD, Peroxidase) ausgenutzt werden. Damit soll eine Stimulierung der ATP- Synthese möglich sein.

Gemäß der DE 100 61 419 A1 sollen Proteinhydrolysate zur topischen Restrukturierung insbesondere keratinöser Fasern wie Wolle, Pelz, Haar eingesetzt werden. Gemäß der EP 1635776 (WO 2004/105716) werden spezielle Lipidtransferproteine in Kombination mit Lipiden für die verbesserte externe Lipidversorgung von Haut und ihrer Anhangsgebilde vorgeschlagen.

Die EP 1781330 (WO2006/02127 A) betrifft ein Modul aus gefäßbeeinflussenden Substanzen wie Aktivatoren (z. B. Ginkgo, Arginin), Protektoren wie z. B. B- Vitamine sowie Mitteln für die Energieversorgung. Dieses Modul wird zusammen mit anwendungsbezogenen Wirkstoffen kombiniert und führt zu einer selektiv verbesserten Wirkung wie z. B. bzgl. der Haut bei hautwirksamen aufbauenden Zusatzsubstanzen (z. B. Biotin, Zink-Verbindungen).

Die o. g. Kombinationen betreffen insofern den verbesserten Aufbau der Gewebestruktur durch Beeinflussung mittels anti- oxidativer oder gefäßaktivierender Maßnahmen. Es wäre indes wünschenswert, wenn nicht nur ein verbesserter Aufbau der Textur lebenden Gewebes erreicht werden könnte, sondern insbesondere dem Abbau dieser Textur wirksam entgegengewirkt werden könnte.

US 2006/198810 A1 beschreibt Zubereitungen mit Traubenkernöl, Angelika-Wurzel-Extrakt, Glucosamin, Niacin zur Verbesserung der Kollagen- und Lipidstatus' der Haut.

EP 1 609 461 A1 betrifft topische Zubereitungen mit Grüntee, Glucosamin, Tocopherol.

WO02/076423 A2 offenbart topische Zubereitung zur Hautstrukturverbesserung mit Glucosaminen, Terpenalkohol, ggf. Niacin, Grüntee und Tocopherol.

D4 FR 2 573 651 A1 betrifft fluide Hautbehandlungsprodukte mit ätherischen Ölen und essentiellen Fetten sowie wahlweise wie Extrakte aus Hibiskus, Ginseng, Calendula, Rosmarinöl zur Wiederherstellung des dermalen Hautgleichgewichts.

Die Online Verkaufs- und Meinungs-Plattform "Mintel" offenbart diverse Produkte zur Anwendung für die Haut oder oral mit unterschiedlichen Zusammensetzungen: Database GNPD (online) Mintel, December 2009 (2009-12), anonymous: "Rejuvenating Eye cream", database accession no. 1282287 beschreibt eine Augencreme mit Isoflavonen und AHA's sowie wahlweise auch weiteren Vitaminen (A,C,E) Traubenkern- und Beifußextrakt sowie essentiellen Fettsäuren (z. B. Borretschöl, siehe Rezeptur),sowie Calendula, Echinacea, Melisse u.a.

Database GNPD (online) Mintel, November 2009 (2009-11), anonymous: "Facial moisturizing mask", database accession no. 1207851 betrifft eine Feuchtigkeitsmaske, deren besondere Wirkung auf Hyaluronsäure und Aloe Vera Extrakt zurückzuführen sein soll. Weitere Inhaltsstoffe können gewählt werden aus Kamelie, Hibiskus-Extrakt, Calendula.

Database GNPD (online) Mintel, October 2009 (2009-10), anonymous: "Shampoo", database accession no. 1190387 betrifft ein Shampoo mit besonderer Wirkung aufgrund vorhandener ätherischer Öle wie Pfefferminzöl, Rosmarinöl, sowie Ginseng und weiterhin Feuchtigkeitsspendender Substanzen wie Glycerin zur Hydratisierung sowie Centela, Quinoa, Vitamin A. Damit soll Volumen in das Haar gebracht werden, siehe Titel und Blatt 1, Mitte unter "Product Description". Weitere Bestandteile können gewählt werden aus Kamelie, Hibiskus, Calendula.

Database GNPD (online) Mintel, September 2009 (2009-09), anonymous: "Night cream with cranberry juice", database accession no. 1173933 betrifft eine Nachtcreme mit besonderen Effekten durch die Anwesenheit von Cranberry. Hinzu kommen noch Harnstoff, sowie Vitamine wie Vitamin A, E, C, essentielle Fettsäuren wie Sonnenblumenkernöl. Weitere Wirkstoffe können gewählt werden aus Echinacea, Calendula, Preiselbeerextrakt.

Database GNPD (online) Mintel, June 2009 (2009-06), anonymous: "C is a smooth Body Moisturizing Lotion", database accession no. 1110778 D9 beschreibt eine Feuchtigkeitslotion, deren Wirkung im Wesentlichen auf Karottensamenöl, Karottensaft - also Vitamin A-, Meersalz, Melone, Süßkartoffel zurückgeführt wird. Weitere Inhaltsstoffe können gewählt werden aus Braunalgen, Olivenöl, Calendula, Niacin. Database GNPD (online) Mintel, June 2008 (2008-06), anonymous: "Healthy skin with Greens + Drink powder", XP055668868, database accession no. 919500 betrifft ein Trinkpulver mit vor allem Grünteeextrakt sowie als Zuckerersatzstoff Stevia. Aus der Inhaltsliste ergeben sich diverse weitere unterschiedlichste Komponenten, wie Reispulver, Licorine Extrakt, Acerolabeerextrakt, Vitamin C usw.

Database GNPD (online) Mintel, September 19, 2008 (2009-09-19), anonymous: "Liquid dietary supplement", XP055668861 database accession no. 975872 beschreibt ein anti-Aging Getränk als sog. ,cellulare antioxidant' mit insbesondere Reservatrol (antioxidatives Diphenol), und einem Gemisch aus Fruchtauszügen zur Herstellung einer Super- Frucht- Saftmischung. Weitere Inhaltsstoffe können gewählt werden aus Ginkgo-, Ginseng-, Traubenextrakt.

Database GNPD (online) Mintel, April, 02 2009 (2009-04-02), anonymous: "Dietary supplement", XP055668873, database accession no. 1081422 betrifft ein Nahrungsergänzungsmittel mit Vitaminen Antioxidantien und Teeextraktmischungen (aus Grüntee, Weißtee, Schwarzem Tee).

Database GNPD (online) Mintel, September 22, 2003 (2003-09-22), anonymous: "Woman's Blend Multi-Vitamin Supplement", XP055668870, database accession no. 10148652 betrifft ein Multi-Vitamin-Multi Mineralprodukt speziell für Frauen mit einer Mischung aus Vitaminen, Mineralien, Kräuterextrakten.

### Aufgabe der Erfindung

Aufgabe vorliegender Erfindung ist es daher, eine Zubereitung bereitzustellen, mit welcher dem Abbau der Textur lebenden Gewebes unter gleichzeitigem Aufbau (Forming) derselben entgegengewirkt werden kann.

### Lösung der Aufgabe

Diese Aufgabe wird erfindungsgemäß dadurch gelöst, dass man Zubereitungen zur Stabilisierung der Textur lebender Gewebe, insbesondere menschlicher Haut und ihrer Anhangsgebilde wie z.B. Haarfollikel und Nagelbett sowie Bindegewebe bereitstellt, welche eine Kombination aus 4 Grundbestandteilen, nämlich aus A) Inhibitoren des Abbaus der Gewebematrix durch Collagenase, Elastase und Hyaluronidase sowie B) Stimulatoren des Gewebematrix-Aufbaus, und vorzugsweise zusammen mit C) Aktivatoren der Blutgefäß Funktion sowie D) Aktivatoren der Nervenfunktion (neurotrope Substanzen) aufweisen. Überraschenderweise kann mit einer derartigen Kombination in synergistischer, wirkungssteigernder Weise degenerativen Prozessen des Abbaus der Gewebetextur entgegengewirkt werden. Die Substanzen A) werden ausgewählt aus A1) aus Terpenoiden wie Sesqui- und Triterpenhaltigen Pflanzenextrakten; ferner aus A2) Phytoaciden Substanzen wie auch phytoiden Fettsäure-haltigen Pflanzenstoffen und Kombinationen aus A1), A2), welche Substanzen A) Inhibitoren der Collagenase/ Elastase und / oder Hyaluronidase darstellen, nämlich aus Kurkuma bzw. Kurkuma- Extrakt, Weihrauchextrakt, Persimone, Teufelskrallenwurzel- Extrakt, Olivenöl, Schafgarbe, Rhabarber, Braunalgen / - extrakt oder Grüntee oder Mischungen hiervon. Die Substanzen B) werden vor allem ausgewählt aus säuregruppenhaltigen / säureartigen Mitteln wie (Extrakte / Pulver aus) Preiselbeerenextrakt; Hibiscus-Extrakt; Morinda citrifolia (Nonifrucht); Granatapfelschalenextrakt; Paprikapulver., Substanzen der Gruppe C) werden gewählt aus Calendula-Blütenextrakt, Tagetesblütenextrakt, Weiße- Lilien- Extrakt; Tomatenextrakt; Niacinr Substanzen der Gruppe D) sind B- Vitamine wie erläutert. Mittels derartiger Kombinationen in geeigneten galenischen Formulierungen zur systemischen und topischen Anwendung wird überraschender Weise ein synergistisch wirkendes Mittel gegen die degenerativen Mechanismen der Gewebetextur - Veränderung bis hin zur Zerstörung erhalten, ohne dass dazu weitere spezifische Wirkstoffe zwingend enthalten sein müssten. Dabei werden topische Applikationsformen und orale Darreichungsformen kombiniert verwendet. So eignen sich derartige Zubereitungen vor allem auch zur Verbesserung der Funktion und somit des Erscheinungsbildes und der Textur von Haut- oder Bindehautgewebe mit z. B. altersbedingter, traumatisch oder krankheitsbedingter verstärkter Abbaufunktion. Diese Verbesserung ist Folge des erfindungsgemäßen Texturaufbaus bzw. der Texturneustrukturierung.

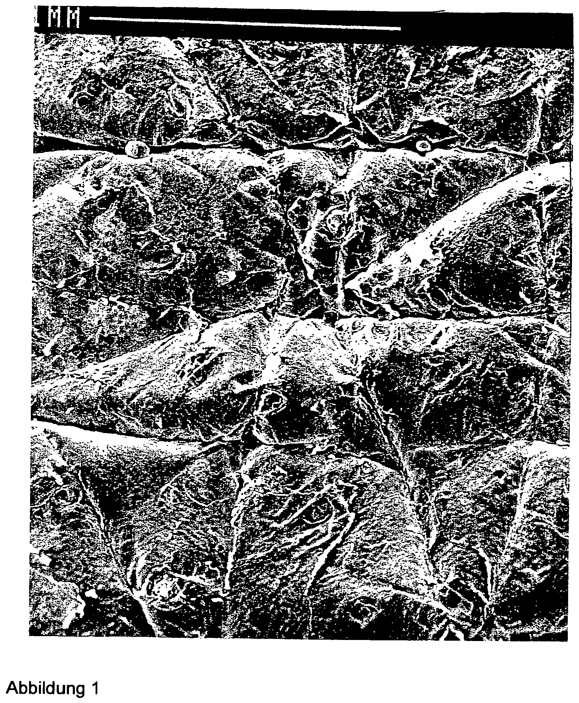

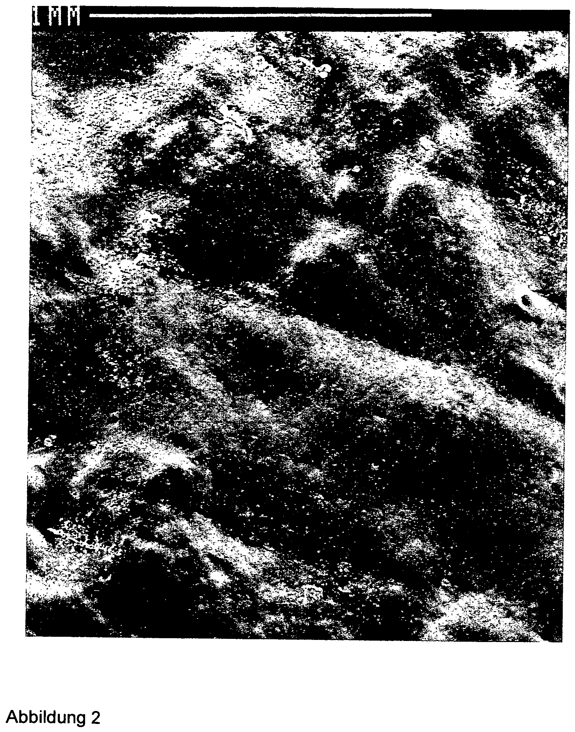

### Erläuterung der Abbildungen 1, 2

Die aus Anwendungsbeispiel II (Hautbehandlung mit erfindungsgemäßen Zubereitungen / Kontrollzubereitungen) erhaltenen Ergebnisse hinsichtlich der Hauttextur sind in Abbildung 1 (Kontrolle) und Abbildung 2 (erfindungsgemäße Kombination Kapsel II + AntiAge Creme) dargestellt und zeigen im Auflicht - Mikroskop in ca. 100-facher Vergrößerung vor (Abb.1) bzw. nach 52-tägiger Anwendung (Abb.2) die Verbesserung von Textur und Erscheinungsbild der Haut nach der erfindungsgemäßen Anwendung.

Nähere Erläuterung erfindungsgemäßer Ausführungsformen Erfindungsgemäße Zubereitungen enthalten als ersten wirksamen Bestandteil A) ein oder mehrere Inhibitoren des Proteinabbaus (des Gewebematrixabbaus), insbesondere Inhibitoren der (hydrolytischen Enzyme) Elastase, Collagenase, und / oder Hyaluronidase. Derartige Wirkstoffe A) sind vor allem ausgewählt aus der Gruppe, umfassend:
A1)Terpenoide, d.h.Terpen-, insbesondere Sesqui-/Triterpenoid - haltige Substanzen wie Kurkuma ( Gelbwurz), Weihrauch (Boswellia), Teufelskrallenwurzel, Grüntee,; oder
A2)Phytoacide Komponenten wie Phytosäurehaltige, z. B. auch Fettsäurehaltige Pflanzenstoffe wie Olivenöl, , Persimone (Kaki bzw. Sharonfrucht oder Blätter, mit Gallensäurederivaten), Braunalgen. oder Kombinationen aus A1),A2).

Die Wirkstoffangabe bezieht sich dabei auf bekannte Weise erhältliche Extrakte bzw. auf isolierte Substanzen sofern vorhanden wie z. B. ätherische Öle (erhältlich z. B. durch Wasserdampfdestillation), oder Pulverextrakte (erhältlich aus getrockneten Blättern, Zweigen wie, Kurkuma, Tee), oder alkoholische Auszüge oder auch Konzentrate, beispielsweise von Beeren, oder auch Pulver z. B. aus Wurzeln, oder isolierte Wirkstoffe, die dem Fachmann bekannt sind. Erfindungsgemäße Substanzen (Wirkstoffgruppe) A) mit deren Aktivitäten sind in Tabelle 1 angegeben, wobei Tabelle 1a) Extrakte /Auszüge / Pulver darstellt Tabelle 1a): Wirkstoffgruppe A (A1,2): Extrakte / Pulver / Auszüge Tabelle 1a zeigt als Inibitoren der Elastase (EI), Collagenase (Cl) und / oder Hyaluronidase (HI) dem Fachmann bekannte natürliche Wirkstoff-Gemische aus Phytodrogen z.B. als wässrig / organische Extrakte aus Blättern, Früchten, Stamm, Wurzeln, Blüten oder als gepresste oder destillierte Öle. Dabei liegen die Droge-Extrakt Verhältnisse üblicherweise zwischen 1:1 und 15:1. Spezial-Extrakte mit Droge-Extrakt Verhältnissen >15:1 können die Wirkung verstärken.

**Tabelle 1a**

| Substanz | Inhibitor der Elastase | Inhibitor der Collagenase | Inhibitor der Hyaluronidase |
|---|---|---|---|
| Kurkuma (curcuma longa) | + | + | + |
| Grüntee (Camelia sinensis) | + | | |
| Persimone / Kaki / Sharon (Diospyros kaki) | + | + | |
| Schafgarbe (Achilla millefolia) | | | |
| Braunalge (Ecklonia cava) | | + | |
| Olive (Olea europaea) | + | | |
| Sharon blätter | | | |
| Weihrauch (Boswellia sacra) | + | | |
| Teufelskralle (Harpago-Phytum procumbens) | + | | |

Besonders bevorzugte Substanzen A) sind ausgewählt aus Elastase- Inhibitoren, vor allem Ölbaumfrucht (Olivenöl, Ölsäure), Grüntee, / Extrakte. Weiterhin besonders bevorzugt sind Elastase- und Collagenase-Inhibitoren wie vor allem Weihrauch -Extrakt, Teufelkrallewurzel- Extrakt, und Braunalgen / Braunalgenextrakt. Oder auch Elastase / Collagenase- Inhibitoren wie in Tabelle 1a) angegeben wie Kurkuma, , Kaki etc. Es können auch einzelne Mischungen derartiger Extrakte verwendet werden, wie z. B. Mischungen mit Grünteeextrakt. Weiterhin bevorzugt sind Hyaluronidase- Inhibitoren, besonders Kurkuma, Persimone (Kaki/ Sharonfrucht, Blätter),.

Ganz besonders bevorzugt ist Kurkuma bzw. Kurkuma- Extrakt, Weihrauchextrakt, und Teufelskrallenwurzel- Extrakt, Oliven (Öl, Extrakt), Schafgarbe, Braunalgen-/ Extrakt oder Grüntee, oder Mischungen hiervon. Vor allem bevorzugt ist Kurkuma-Wurzel-Extrakt oder Braunalgen / Braunalgenextrakt oder Mischungen hiervon oder hiermit mit anderen A-Komponenten.

Zu dem in den erfindungsgemäßen Zubereitungen enthaltenen wirksamen Bestandteil Wirkstoffgruppe B) gehören nachfolgend beispielhaft folgende Substanzen (Tabelle 2), wobei die Bildung von Collagen und Proteoglykanen im Vordergrund steht:

**Tabelle 2**

| Extrakte / Auszüge/ Substanzen und Substanzgemische der Gruppe B |
|---|
| - Malvengewächse (Hibiscus) |
| - Heidelbeere (Vaccinium myrtillus) |
| - Preiselbeere |
| - Maulbeerbaum Extrakt (Morinda itrifolia, Nonofrucht) |
| **- Granatapfel (Punica granatum)** |
| **- Paprika (Capsicium annum)** |

Der in den erfindungsgemäßen Zubereitungen enthaltene wirksame Bestandteil B) ist insbesondere ausgewählt aus säurehaltigen / säureartigen Substanzen wie Vitamin C, Hibiscus - Extrakt (Eibisch, Chinesischer Eibisch, Roseneibisch, Straucheibisch), Heidelbeeren, Preiselbeeren - Extrakten, Paprikapulver, ggf. auch Granatapfelschalen- Extrakt, insbesondere in Kombination mit anderen Substanzen C) oder Substanzen der Gruppe D). Derartige Substanzen weisen im wesentlichen Fruchtsäuren wie Hydroxysäuren, z. B. Ascorbin-, Zitronen-, Äpfel-, Weinsäure ggf. neben phenolischen Komponenten, Phytosterolen auf. Besonders bevorzugt sind Hibiscus- Extrakt, Paprika, Heidelbeere, oder auch Granatapfelschalen-Extrakt. Bestandteile C) werden erfindungsgemäß z. B. ausgewählt aus Niacin, , Calendula (Calendula officinalis, Ringelblumenextrakt z. B. in Form des Öls aus Blüten, enthaltend Carotinoide, Flavoxanthine, Lutein), Tagetesblüten- Weiße- Lilien- Extrakt, Tomatenextrakt (enthaltend Lutein).

Besonders bevorzugt ist Calendula-Blütenextrakt bzw. ÖI- Extrakt, Niacin, Tomatenextrakt oder Mischungen hiervon. Ebenfalls bevorzugt ist Niacin oder Mischungen hiervon, vor allem auch im Gemisch mit Ringelblumenextrakt, oder Mischungen hiervon.

Als neurotrope Mittel der Substanzgruppe D) können beispielsweise die B- Vitamine, eAuszüge aus Getreide wie z.B. Buchweizen- eingesetzt werden. Sie können insbesondere ausgewählt sein aus Vitamin B2, B5, B6, B12, B1 (z.B. Thiaminnitrat (B₁), Getreideauszügen wie Buchweizenextrakt oder Mischungen einzelner Extrakte wie genannt. Vorzugsweise wird B5, B1 oder Mischungen hiervon gewählt. Vor allem geeignet ist B5, z. B. Calciumpanthotenat z. B. auch im Gemisch mit einem oder mehreren Vitaminen, ausgewählt aus B1, B2, B6, B12, auch insbesondere im Gemisch mit Getreideauszüg(en).

Besonders bevorzugte Zubereitungen enthalten einen oder mehrere Elastase- Inhibitoren A) wie in Tabelle 1 genannt; einen oder mehrere Elastase- Inhibitoren A) wie in Tabelle 1 genannt, Hibiscus- Extrakt, , Paprikapulver, oder Mischungen hiervon als Komponente B), vorzugsweise diese Komponenten in Kombination mit Calendula oder Tomatenextrakt, Niacin oder Mischungen hiervon (Komponente C), und / oder Vitamin B5 (z. B. Ca- Pantothenat), Getreideauszüg(e), oder Vitamin B1, oder Mischungen hiervon mit B1, B2, B6, B12 als Bestandteil D). Als Komponente A) wird insbesondere hier Teufelskrallewurzelextrakt, Weihrauch- Extrakt, Olive(nöl), / Extrakte, Braunalgen/-extrakt oder Grüntee oder Mischungen hiervon wie eingesetzt.

In einer anderen Ausführungsform werden als Komponente A) vor allem Hyaluronidase- Inhibitoren eingesetzt, welche ausgewählt werden aus Kurkuma bzw. Extrakten, , einzeln oder im Gemisch.

Es ist weiterhin von Vorteil, wenn als Komponente A) derartige bevorzugte Hyaluronidase- Inhibitoren (vor allem Kurkuma bzw. Extrakten hiervon, , einzeln oder im Gemisch) eingesetzt werden zusammen mit Elastase- / Collagenase- Inhibitoren wie in Tabelle 1 genannt, vor allem den oben genannten bevorzugten Inhibitoren wie Teufelskrallewurzelextrakt, Weihrauch- Extrakt, Olive(nöl), -/ Extrakte oder Mischungen hiervon.

Erfindungsgemäße Kombinationen umfassen somit Substanzen der Gruppen A + B +C+D.

Es kann weiterhin von Vorteil sein, wenn in erfindungsgemäßen Zubereitungen neben den Komponenten A), B) Niacin als Komponente C), insbesondere in Kombination mit o. g. bevorzugten Elastase- / Hyaluronidase- Inhibitoren vorhanden ist. Dabei kann Komponente B) vor allem gewählt werden aus Hibiscus- Extrakt oder Paprikapulver, , oder Ringelblumenextrakt oder Mischungen hiervon, Bevorzugt ist Hibiscus- Extrakt oder Gemische hiermit wie genannt. Dazu kann auch bevorzugt Niacin oder Tomatenextrakt kombiniert werden.

Eine weitere bevorzugte Ausgestaltung vorliegender Erfindung betrifft Kombinationen, enthaltend Niacin, Paprikapulver, Grünteeextrakt, ein oder mehrere Vitamin B-Substanzen oder Derivate hiervon wie genannt (B1, B2, B5 (z. B. Calciumpantothenat), B6, B12), Getreideauszüge oder Gemische hiervon.

Die erfindungsgemäß kombinierten Extrakte oder Öle liegen vorzugsweise in einer Gesamtmenge A+B + C+D von 0,7 bis 70 Gew. %, vor allem 1 bis 70 Gew. %, vorzugsweise 3- 50 Gew. %, oder auch 5- 25 Gew. %, bezogen auf die Gesamtzubereitung vor. Den Rest (z.B. 99,3 bis 30 Gew.%) bilden dann die für die galenische Zubereitung erforderlichen Zusatzstoffe, ggf. einschließlich Zusatzwirkstoffe.stanzen Die angegebenen Mengenbereiche sind jedoch variabel., Dem Fachmann sind die jeweiligen Anteile der wirksamen Bestandteile geläufig, so dass dies auf bekannte Weise an die galenische Ausführungsform angepasst werden kann.

### Bevorzugte Ausführungsformen

Bevorzugte Zubereitungen umfassen z. B. Kombinationen gemäß Tabelle 3:

**Tabelle 3:**

| Wirkstoffgruppe A) | Wirkstoffgruppe B) | Wirkstoffgruppe C) | Wirkstoffgruppe D) |
|---|---|---|---|
| Teufelskrallenwurzel-Extrakt | Hibiscus-Extrakt | Ringelblumenextrakt | Ca-Pantothenat |

Eine weitere bevorzugte Kombination umfasst Zubereitungen, enthaltend jeweils aus der Gruppe A, B, C, D mindestens einen Vertreter, ausgewählt aus Weihrauchextrakt, Teufelskrallenwurzelextrakt, Wacholder Extrakt, Ölbaumfruchtextrakt, Persimone, Braunalgen /-extrakt oder Mischungen hiervon sowie Tomatenextrakt, Niacin , Hibiscus-, Ringelblumenextrakt, Buchweizenextakt, Calciumpantothenat, oder Mischungen hiervon.

Eine andere bevorzugte Kombination umfasst Zubereitungen, enthaltend Kurkuma-Wurzel- Extrakt, Weihrauch- Extrakt, oder Mischungen hiervon, insbesondere zusammen mit Hibiscus- Extrakt und / oder Heidelbeerextrakt, Niacin oder Calendulablüten-Extrakt und Calciumpantothenat.

Derartige Zubereitungen weisen Zusatzstoffe auf, welche für die galenische Darreichungsform, insbesondere die systemisch / enterale / orale Form wie Tabletten, Dragees, Kapseln, geeignet sind. So umfassen die beschriebenen Zubereitungen vorzugsweise 0,7 bis 70 Gew. %, vorzugsweise bis 60 Gew. % der Kombination A+B+ C+D und 99,3 bis 30 Gew. %, vorzugsweise bis 80 Gew. % Zusatzstoffe, ggf. Zusatzwirkstoffe. Als galenische Zusatzstoffe kommen vor allem Sprengmittel, Füllstoffe, Fließmittel, Überzugsmittel, Farbstoffe, Kapselmaterialien infrage.

In topischen Verabreichungsformen wie vorstehend genannt können vor allem 1 bis 40 Gew. % der Kombination vorliegen. Als (galenische) Zusatzstoffe sind hier insbesondere Lösungsvermittler wie Wasser, aliphatische Alkohole u. ä. oberflächenaktive stabilisierende Substanzen wie Kolloide, emulgierende Mittel, sowie Farb-Parfümstoffe, Lipide (Fette (Öle), vorhanden.

### Gewinnung der Wirkstoffe

Die erfindungsgemäß einzusetzenden Wirkstoffe werden auf bekannte Weise gewonnen und in dem Fachmann bekannten Mengen eingesetzt:
Ätherische Öle wie, Kamelienöl, können durch Wasserdampfdestillation aus den Grundstoffen erhalten werden. Die Öle werden in üblichen Mengen eingesetzt.
Durch Lösungsmittelextraktion (z. B. mit Alkoholen, Triglyceriden oder Kohlenwasserstoffen, Wasser, Gemischen hiervon) werden Pflanzenextrakte zum direkten Einsatz erhalten.

Pulverextrakte z. B. aus Grüntee, oder Wurzelextrakte wie solche der Teufelskralle, können nach Trocknen der Blätter oder Zweige durch Extraktion mit Lösungsmitteln wie Wasser, Alkohol, organische Lösungsmittel oder Gemischen hiervon und nachfolgender Trocknung gewonnen werden. Derartige Pulver werden je nach Wirksubstanz in bekannten Mengen wie z. B. 5-100 mg / Wirkeinheit, z. B. 10-80 mg Kurkuma - Extrakt verwendet.

Braunalgenextrakt wird gewonnen durch Extraktion mit Wasser bei erhöhter Temperatur, z. B. 30-50° C. Bevorzugt werden Braunalgen der Bretonischen Küste eingesetzt. Die Reinigung erfolgt z. B. durch Ultrafiltration und anschließende Trocknung zum Pulverextrakt.

Beeren, wie Heidelbeere / Blaubeere werden insbesondere auch in Form von Konzentraten eingesetzt.

Blütenextrakte, wie solche aus Ringelblume, können durch Extraktion mit Alkohol, vor allem Ethanol, oder Alkohol / Wasser- Gemischen und nachfolgende bekannte Aufarbeitung gewonnen werden. Diese werden vorzugsweise in Mengen von 10-150 mg verwendet.

Hibiscus- Extrakt, kann als Pulver eingesetzt werden oder als Extrakt wie Grüntee u. ä. gewonnen werden.

Ölbaumfrucht- Extrakt bzw. Olivenöl wird durch Pressung der Frucht bei nicht erhöhten Temperaturen gewonnen.

Vitamin- B- Derivate sind ebenfalls bekannt und erhältlich als solche. Sie können in z. b. für die systemische Verabreichung bekannten Mengen eingesetzt werden wie z. B. Folsäure: 0,2 bis 5 mg, bevorzugt 0,2 bis 0,8 mg, vor allem 0,4 mg; Vitamin B6 (Pyridoxin): 2,0 bis 25 mg; bevorzugt: 2,0 bis 10 mg, insbesondere bis 6 mg, und ganz besonders 2 mg; Vitamin B12 (Cyanocobalamin): 0,003 bis 0,6mg, insbesondere bis 0,1 mg, vor allem 0,006 mg; Vitamin B5, z. B. Ca- Pantothenat: 5-50 mg; Riboflavin (B₂): 5-50, z. B. 25 mg; Thiaminnitrat (B₁) 1-50 mg / pro Tablette oder Dragee / Kapsel.

Zur topischen Anwendung eignen sich entsprechende Mengen der Kombinationswirkstoffe.

### Verabreichungsformen und Herstellung der Zubereitungen

Die erfindungsgemäßen Kombination eignen sich für die systemische, insbesondere enterale Verabreichung und werden auch in topischen Darreichungsformen eingesetzt.. Sie können insofern mit geeigneten Zusatzstoffen, ggf. einschließlich Zusatzwirkstoffen (z.B. gemäß Stand der Technik) wie z. B. Kieselgel, Aminosäuren (z.B. Methionin oder Cystein, L-Cystein), Mineralien, Spurenelemente (z.B. Magnesium, Zink, Eisen, Kupfer bzw. bekannte geeignete Derivate hiervon wie Salze), Vitamine (z.B. Biotin (Vitamin H), Vitamin A, Vitamin E, bzw. bekannte Derivate der Vitamine wie Acetate, usw.), ungesättigte, omega 3-/ 6-Fett-säuren u.ä. kombiniert werden.

### Systemische Verabreichungsformen:

Hierfür sind Tabletten, Kapseln oder Dragees geeignet. Dazu verwendbare (auch pharmazeutisch zulässige) Träger können ausgewählt werden aus Tablettierhilfsstoffen wie Sprengmittel, Füllstoffe, Fließmittel wie z. B: Hydroxypropyl/ - methylcellulose und Analoga, Stärke (Weizen, Korn, Reis, Kartoffel-), Gelatine, Sucrose, Natriumcarboxymethylcellulose, pulverisierte Cellulose, Polyvinylpyrrolidon, Sili-ciumdioxid, Povidonen und Analoga, Titandioxid, Calciumphosphat, Mannitol, Polyethylenglykol, Glycerin, Sorbitol, kolloidale Kieselsäure, Propylenglykol, Aluminiumstearat u.a., Farbstoffen. So können Weich- oder Hartgelatine- Kapseln, Dragees oder Tabletten hergestellt werden, insbesondere mit Hilfsstoffen ausgewählt aus Lactose, Saccharose, Sorbit, Talkum, Stearinsäure, Magnesiumstearat, Gummi arabicum, Kartoffelstärke, Gelatine, PVP, Glycerin, Hydroxypropylmethylcellulose, Maltodextrin, Konservierungsmitteln sowie üblichen Materialien für Überzüge wie PEG 6000, Maisstärke, Zucker, Talkum, Farbstoffen in an sich bekannter Weise. So kann bei oraler Verabreichung die Zubereitung beispielsweise mit Lactose, Saccharose, Stärkepulver, Celluloseester oder Alkansäuren, Cellulosealkylester, Talk, Stearinsäure, Magnesiumstearat, Magnesiumoxid, Natrium- und Calciumsalzen von Phosphor- und Schwefelsäuren, Gelatine, Akaziengummi, Natriumalginat, Glykolen, Polyvinylpyrrolidon und/oder Polyvinylalkohol zunächst vermischt und sodann in bekannter Weise tablettiert oder verkapselt werden. Diese Methoden sind allgemein bekannt und z.B. im aktuellen Europäischen Arzneibuch beschrieben.

Auf diese Weise können pharmazeutische Zubereitungen oder auch Supplemente (nicht therapeutisch) wie Nahrungsergänzungsmittel, Diätikum, ergänzende bilanzierte Diät erhalten werden. Als Supplement können auch Nahrungskomponenten, ausgewählt aus Aminosäuren, Kohlenhydraten, Fetten, die für die menschliche oder tierische Nahrung geeignet sind, z. B. Kohlenhydrat - Protein- Mischungen, u.a. gewählt werden. Je nach Wahl derartiger Vertreter der genannten Einzelkomponenten, können wie erwähnt Riegel, Flüssigkeiten, Drinks, z. B. Kraftdrinks (vorzugsweise nicht sirupös/ wässrig sirupös), Brausetablette oder auch bilanzierte diätetische Ergänzungsmittel dieser Art hergestellt werden. Dazu wird die wie beschrieben bereitete Zubereitung mit einer oder mehrere Nahrungskomponenten auf an sich bekannte Weise gemischt, und die gewünschte Form erhalten durch übliche Herstellungsverfahren.

### Topische Verabreichungsformen:

Für die topische Verabreichung eignen sich insbesondere Fluids oder emulsionsartige Zubereitungen. Hierbei finden flüssige Träger wie Wasser, Alkohol (z. B. Ethanol) oder Mischungen hiervon, ggf. zusammen mit Gelbildnern oder Viskositätsregulatoren (Stabilisatoren) Verwendung. Emulsionsartige Produkte weisen zusätzlich eine Ölphase aus Lipiden und ggf. stabilisierende / emulgierende Zusätze und / oder pflegende Wirkzusätze auf.

Als Lipide können insbesondere verwendet werden: natürliche Öle wie Sojaöl, Kokosnussöl, Jojobaöl, Avocadoöl, Erdnussöl, Traubenkernöl, Sonnenblumenöl, Borretschöl, Sesamöl, Olivenöl, Aprikosenkernöl, Maiskeimöl, Weizenkeimöl, Walnussöl, Palmöl, Macadamianussöl, Palmkernöl, Haselnussöl, Hagebuttenkernöl, Baumwollsamenöl, Mandelöl, Mandelbutter, Lecithin, Distelöl, Macademiaöl, Papayaöl oder auch übliche Lipide wie Fettsäureester wie mittelkettige (z. B. C₈₋₁₂) Triglyceride, mittelkettige (C₈₋₁₂) mittelkettige Propylenglyokolester wie z. B. Fettsäureester, z.B. C₂₋₁₈-Alkoholfettsäure-ester wie Isopropylfettsäureester (Palmitat, Myristat, Isostearat, Oleat), Decyl Oleate, Hexyl Laurate, C12-15 Alkyl Benzoate, Dicaprylyl-Carbonate sowie verzweigte Fettsäureester wie Cetearyl Octanoate, Di-n-Butyl-adipate. Weiterhin sind C₈₋₂₂- Fettalkoholether wie Dicaprylyl Ether oder Fettalkoholester wie C₁₂₋₁₃- Alkyl Lactate, insbesondere Glyceride (C₁₂₋₂₂Fettsäure-Glyce-ride) wie Triglyceride, insbesondere mittelkettige (Neutralöle) wie Caprylic/Capric- Triglyceride sowie deren Polyolester wie Propylene Glycol Dicaprylate/ Dicaprate u. a. Emulgierende (Stabilisierende) Zusätze können gewählt werden z.B. aus Zuckerestern von gesättigten, ungesättigten, partialgesättigten C₁₂-C₁₈-Fettsäuren. Als Kohlenhydrate kommen dabei Mono-, Di- und/oder Oligo-Saccharide oder auch substituierte, insbesondere nieder- Alkyl-, vor allem CH₃substituierte Derivate hiervon infrage. Insbesondere bevorzugt sind Glucose, Methylglucose, Fruktose, Saccharose. Besonders geeignet sind Glukose, Methylglucose und Saccharose. Als Fettsäuren sind hier vor allem organische aliphatische C₁₂-C₁₈-Carbonsäuren geeignet. Hierzu gehören insbesondere Caprylsäure, Caprinsäure, Laurinsäure, Myristinsäure, Palmitinsäure, Stearinsäure, Ölsäure und auch Mischungen dieser. Alternativ können auch Zuckerether von gesättigten, ungesättigten, partial gesättigten C₈-C₂₀-Fettalkoholen verwendet werden, nämlich Glucoside aus den o. g. Zuckern und ein- oder mehrwertigen Alkoholen mit C₈-bis C₂₀ Kettenlänge. Diese emulgierenden Zusätze können beispielsweise in einer Menge von 0,3 bis 10 Gew.% eingesetzt werden. Alternativ wären auch polyoxyethylierte C₈-C₂₀ Alkohole oder polyoxyethylierte C₁₂-C₁₈- Fettsäuren einsetzbar. Je nach Alkylkettenlänge bzw. Ethoxylierungsgrad (z. B. 2-150, vor allem 2-40 EO) werden O/W- bzw. W/O Emulgatoren erhalten wie Polyoxyethylenglykol-20-400-monostearat, oleylether,-monolaurat,-sorbitan-mono-laurat, -sorbitantristearat. Andere Emulgatoren können z. B. aus Sorbitan Estern wie Sorbitan Oleate, Sorbitan Stearate, Sorbitan Sesquioleate, Sorbitan Isostearate, Sorbitan Tristearate oder auch Glycerin-Ester wie Glyceryl Ricinoleate, Glyceryl Laurate, Glyceryl Dioleate S/E; Polyolester wie Glycol Oleate, Glycol Ricinoleate, Glycol Dilaurate,Propylenglykol Laurate; Glucose-Derivate: Glucose Ester wie Methyl Glucose Dioleate, Methyl Glucose Isostearate; gewählt werden, wobei es sich bei letzteren insbesondere um W/O- Emulgatoren handelt. Auch eignen sich bestimmte Kolloide, insbesondere Hydrokolloide wie Proteinderivate tierischer Natur z. B. Hydrolysate, wie Milchprotein, mit einer höheren mittleren Molmasse , z. B. MW größer 40000 D, wasserlösliche Caseinverbindungen, insbesondere Salze von Casein, wie Natriumcaseinat mit relativen Molmassen im Bereich von 15000 bis 30000 D, ferner Gelatine (z. B. Typ A oder B), Albumine, oder Mischungen hiervon, oder auch wasserlösliche (Poly)(Meth)Acrylatpolymere wie Carbomere, oder wasserlösliche Cellulosederivate wie Methylcellulose, Hydroxypropy-Icellulose, Hydroxyethylcellulose, Hydroxypropylmethylcellulose, wobei letztgenannte Kolloide eine mittlere molare Masse von mehr als 40 000 D, z. B. Carbomere 1,25 bis **4** Mio. aufweisen können. Derartige Kolloide können auch als Gelbildner verwendet werden in nicht lipidhaltigen Zubereitungsformen.

Die Lipide werden üblicherweise z. B. in Mengen von 2 bis 60 % eingesetzt. Emulgatoren, (Stabilisatoren) können auch kombiniert werden und in Mengen von z. B. 0,5 bis 15 Gew.% vorliegen. Daneben sind auch andere bekannte Stabilisatoren (Konditioniermittel oder Konservierungsmittel) möglich. Als Farbstoffe eignen sich hierfür bekannte Mittel, Parfümstoffe können aus etherischen Ölen oder synthetischen Mitteln gewählt werden. Lösungsvermittler, und weitere Zusätze wie oben genannt sind jeweils in erforderlicher Menge vorhanden.

Pflegende Stoffe können gewählt werden aus Feuchthaltern wie Glycerol, Propylenglykol, Sorbitol, Harnstoff; Cetiol HE, Panthenol (Pantothenylalkohol), Allantoin, Bisabolol, Lecithin (Sojalecithin) sowie Ceramide und Sterole (z.B. Cholesterol) oder Polysiloxanen; ggf. auch Parfümölen, Proteinhydrolysaten insbesondere pflanzlicher Herkunft wie Weizen, Hafer, Gerste, Buchweizen, MW 3500 - 20000 D.

Die topisch verabreichbaren bzw. systemisch verabreichbaren Mittel werden auf übliche, dem Fachmann bekannte Weise hergestellt, wobei die für die jeweils gewünschte Anwendungsform geeignete Zusatzstoffe in bekannter Menge verwendet werden.

### Kombinatorische Anwendung systemisch / topisch

Die erfindungsgemäßen Zubereitungen werden kombiniert angewendet. Für die systemische bzw. enterale Anwendung sind orale Verabreichungsformen besonders geeignet, z. B. in Form von Gelatinekapseln, z. B. Weichgelatinekapsel, sowie Tabletten. Für die topische Verabreichung z. B. durch Auftragen / Einreiben, Sprühen, Massieren, eignen sich Lotionen, Sprays, Cremes, Gele, Fluids, Emulsionen.

Die Zubereitungen werden eingesetzt zur Verbesserung der Textur lebender Gewebe, insbesondere der Haut und ihrer Anhangsgebilde wie Haarfollikel und/oder Nagelbett sowie des Bindegewebes bei Säugetieren, insbesondere bei Menschen. Diese Verbesserung ist Folge des erfindungsgemäßen Texturaufbaus bzw. der Texturneubildung, z. B. in nicht medizinischen oder medizinisch- therapeutischen Anwendungen oder kosmetischen Anwendungen bzw. zur Herstellung derartiger Mittel.

Eine Strukturverbesserung ist vor allem angezeigt bei verlangsamten Aufbauprozessen von Gewebebestandteilen, z. B. aufgrund einer Erkrankung, eines geschwächten physischen Zustandes, eines Traumas, oder auch altersbedingt. Durch die erfindungsgemäße Kombination kann daher insbesondere der altersbedingten, der traumatisch (z. B. durch Verletzung, Schnitt, Verbrennung, Narben), oder auch krankheitsbedingten Abbaufunktion von vor allem menschlicher Haut und Bindegewebe, vor allem zur Verbesserung des Erscheinungsbildes und der Textur von Haut, Bindegewebe, entgegengewirkt werden. Dies wird erreicht durch eine Strukturstabilisierung sowie eine Regenerationsförderung, insbesondere durch die Komponenten A + B, zusammen mit einer Schutzaktivierung von Gefäßen und Nerven durch die Komponenten C bzw. D. Dabei kann ein (wirkungssteigernder) synergistischer Effekt festgestellt werden.

Die Menge und Dosierung der beschriebenen Zubereitung zur Prävention oder Behandlung einer wie oben beschriebenen Gewebetextur hängen bei einer medizinischen Indikation von einer Vielzahl von Faktoren ab, einschließlich dem Alter, Gewicht, Geschlecht und Zustand des Patienten, der Schwere der Störung, der Verabreichungsroute u.ä. Sie kann weitestgehend schwanken. Die erforderliche oder gewünschte Dosis kann auf ein- oder mehrere Male täglich, insbesondere 1 bis 3 mal verteilt werden.

Bei der Verabreichung als Supplement werden hierfür allgemein übliche Mengen vorgeschlagen, welche auf ein- bis mehrmals, insbesondere 1 bis 3 mal oder 1 bis 2 mal täglich verteilt werden können. Diese können in fester Form, z. B. als Tablette, Kapsel, Dragee, oder als Riegel bzw. eine entsprechende Menge gelöst in Säften vorliegen.

Die topische Verabreichung der dazu beschriebenen Formen erfolgt auf die Haut direkt wie oben beschrieben und je nach Erfordernis 1 bis mehrmals täglich. Bevorzugt werden Gele, Fluids und Lotionen eingesetzt. Hierbei sind ganz besonders Produkte geeignet, welche Tomatenextrakt, , Grüntee, Braunalgen / -extrakt, Niacin, Hibiscus-, Ringelblumenextrakt oder Mischungen hiervon sowie Calciumpanthotenat aufweisen. Zusätzlich können hierbei auch vor allem noch Weihrauchextrakt, , Ringelblumen-Extrakt wie beschrieben, Ölbaumfruchtextrakt, oder Mischungen hiervon enthalten sein.

In einer weiteren bevorzugten Ausführungsform werden vor allem Olivenöl mit Paprikapulver (Substanzen A, B), Niacin (Substanz C) kombiniert,. Es liegen die Wirkstoffe A, B . + C +, D jeweils sowohl in topischer als auch in systemischer Verabreichungsform vor, Es kann insofern ein Kombinationszubereitungskit angewendet werden, der eine systemische Verabreichungsform, sowie eine topische Verabreichungsform, umfasst. Als systemische Verabreichungsform eignet sich insbesondere hier ein Dragee oder eine Kapsel (z. B. Gelatinekapsel), als topische Verabreichungsform ein Gel oder eine Lotion.

### Beispielhafte Ausführungsformen

Bevorzugt werden erfindungsgemäß Dragees oder Gelatinekapseln, insbesondere Weichgelatinekapseln oder Tabletten für die systemische Verabreichung zubereitet. Tonika oder Lotionen werden für die topische Applikation bevorzugt. Nachfolgend werden Beispiele für die Anwendung auf Haare bzw. Haarfollikel beschrieben.

### Anwendu ngsbeispiele

### I. Haare

### 1) Erfindungsgemäße Zubereitungen:

Wirkstoffgruppe A:

| Elastase, Collagenase und Hyaluronidase Inhibitoren | Haar Dragee I | Haar Dragee IIA | Haar Tonikum IIB | Haar Dragee III |
|---|---|---|---|---|
| Wachholderbeeröl | 35 mg | --- | 1.3 % | |
| Kurkumawurzel Extrakt 8:1 | 15 mg | 15 mg | --- | |
| Ginseng Extrakt 1o:1 | 20 mg | --- | 1.1 % | |
| Grüntee Extrakt 8:1 | 15 mg | --- | 1.1 % | |
| Efeublätter Extrakt 6:1 | 10 mg | 10 mg | --- | |
| Sharonblätter Extrakt 6:1 | 20 mg | 20 mg | --- | |

| Wirkstoffgruppe B: Stimulatoren des Matrix-Aufbaus | | | | |
|---|---|---|---|---|
| Paprika Extrakt 4:1 | 10 mg | 10 mg | --- | |
| Granatapfelschalen Extrakt 6:1 | 10 mg | --- | 1.5 % | |
| Hibiskusblüten Extrakt 7:1 | 8 mg | 8 mg | --- | |

| Wirkstoffgruppe C: | | | | |
|---|---|---|---|---|
| Gefäßaktive Komponenten | | | | |
| Niacin | 35 mg | 35 mg | | |
| Ringelblumen Extrakt 6:1 | 18 mg | --- | 1.5 % | |
| Tomaten Extrakt 5:1 | 15 mg | 15 mg | --- | |

| Wirkstoffgruppe D: | | | | |
|---|---|---|---|---|
| Neurotrope Komponenten | | | | |
| Calciumpantothenat | 20 mg | --- | 2 % | |
| Folsäure | 400 µg | 400 µg | --- | |
| Pyridoxin | 5 mg | 5 mg | --- | |
| Buchweizen Extrakt 5:1 | 15 mg | --- | 1.2 % | |

### 2) Zubereitungen gemäß Stand der Technik

Ausschließlich Wirkstoffe gemäß Stand der Technik sind in Dragee III enthalten

| | | | | |
|---|---|---|---|---|
| Alpha-Tocopherol-Acetat | 15 mg | 8 mg | 1.2 % | 15mg |
| Biotin | 6 µg | 6 µg | --- | 6 µg |
| Zink-Sulfat | 8 mg | 8 mg | --- | 8 mg |
| Kupfer-Acetat | 500 µg | 500 µg | --- | 500µg |
| Cystin | 320 mg | 320 mg | --- | 320mg |

### 3) Hilfsstoffe zur galenischen Formulierung:

| | | | |
|---|---|---|---|
| Tablettier-Hilfsstoffe, Coating-Hilfsstoffe, Stoffe zur Mikroverkapselung, Farbstoffe, Aromen q.s. | q.s | q.s. | --- |
| Wasser, Äthanol, Propylenglykol, PEG 40, Polysorbat, KonservierungsStoffe, Aromen | --- | --- | q.s. |

### 4) Vergleichsversuche : Anwendung Haare

Ausgehend von orientierenden ex vivo Untersuchungen einer Kombination der erfindungsgemäßen Wirkstoffgruppen in wässrigem Medium am kultivierten Haarfollikel, die eine Zunahme der Haarschaft Elongation um 6 bis 8 % gegenüber der Kontrolle gezeigt hatten, wurden Zubereitungen Dragee I, Dragee IIA + Tonikum IIB im Vergleich zu Dragee III klinisch mittels Phototrichogramm (R. Hoffmann, Eur. J. Dermatol., 2001,11,362-8: Trichoscan: combining epiluminiscence microscopy with digital image analysis for the measurement of hair growth") im Hinblick auf die Haaranzahl, die Anagen- / Telogenrate und die Wachstumsgeschwindigkeit über einen Zeitraum von 150 Tagen an 6 freiwilligen Teilnehmern (weiblich 52-58 Jahre; postmenopausal, Messzeitpunkt: t₁=0 und t₂=150 Tage) ermittelt. Es wurden jeweils 2 Film-Dra-gees bzw. 2x2 ml Haartonikum (wässrig/ org. Essenz) pro Tag verabreicht. Dabei ergaben sich signifikante Verbesserungen der Dichte der Haare, der Anagen / Telogen Rate und der Wachstums-Geschwindigkeit der Haare um 54 bis 93 Prozent je nach gemessenem Parameter zugunsten der erfindungsgemäßen Rezepturen, wie sich aus der nachfolgenden Zusammenstellung (Tabelle 4) ergibt.

**Tabelle 4**

| Zubereitung / Meßparameter | Steigerung der Haaranzahl (%) | Steigerung der Angenrate (%) | Abnahme der Telegenrate (%) | Steigerung der Wachstumsgeschwindigkeit (%) |
|---|---|---|---|---|
| Dragee I | 4.9 | 9.3 | -20.5 | +4.4 |
| Dragee IIA + Tonikum IIB | 5.4 | 8.9 | -19.9 | +4.6 |
| Dragee III (Vergleich) | 2.8 | 5.4 | -13.0 | +2.9 |
| Max. Verbesserung geg. Vergleich | 93% | 72% | 54% | 59% |

### II. Anwendungsbeispiele Haut

### 1) Erfindungsgemäße Zubereitungen:

Wirkstoffgruppe A:

| Elastase, Collagenase und Hyaluronidase Inhibitoren | Anti-Age Kapseln I | Anti-Age Kapseln II | Anti-Age Creme | Anti- Age Kapseln III |
|---|---|---|---|---|
| Rosmarinöl | 30 mg | 15 mg | 1.0 % | --- |
| Kakiblätter Extrakt 7:1 | 25 mg | 25 mg | --- | --- |
| Ingwer-Extrakt 8:1 | 20 mg | 20 mg | --- | --- |
| Teufelskrallen Extrakt 6:1 | 10 mg | --- | 1.2 % | --- |
| Spanischer Pfeffer Extrakt 4:1 | 15 mg | 15 mg | --- | --- |
| Roibos-Tee Extrakt 6:1 | 15 mg | --- | 1.3 % | --- |

| Wirkstoffgruppe B: | | | | |
|---|---|---|---|---|
| Stimulatoren des Matrix-Aufbaus | | | | |
| Noni-Frucht Extrakt 4:1 | 15 mg | 15 mg | --- | --- |
| Granatapfelschalen Extrakt 6:1 | 10 mg | --- | 1.2 % | --- |
| Hibiskusblüten Extrakt 7:1 | 15 mg | 15 mg | --- | --- |

| Wirkstoffgruppe C: | | | | |
|---|---|---|---|---|
| Gefäßaktive Komponenten | | | | |
| Niacin | 35 mg | 35 mg | --- | --- |
| Weiße Lilien Extrakt 5:1 | 18 mg | --- | 1.2 % | --- |
| Tagetesblüten Extrakt 4:1 | 10 mg | 10 mg | --- | --- |

| Wirkstoffgruppe D: | | | | |
|---|---|---|---|---|
| Neurotrope Komponenten | | | | |
| Hafer Extrakt 5:1 | 30 mg | 20 mg | 0,8 | 0,8 % |
| Calciumpanthotenat | 20 mg | --- | 2,0% | --- |
| Thiaminnitrat | 5 mg | 5 mg | --- | --- |
| Folsäure | 400µg | 400µg | --- | --- |

### 2) Zubereitungen gemäß Stand der Technik

Ausschließliche Wirkstoffe gemäß Stand der Technik sind in Kapsel III enthalten.

| | | | | |
|---|---|---|---|---|
| Alpha- Tocopherol-Acetat | 15 mg | 8 mg | 1.2 % | 15 mg |
| Vitamin A Acetat | 0,4 mg | --- | 0,5 % | 0,4 mg |
| Biotin | 6 µg | 6 µg | --- | 6 µg |
| Zink-Sulfat | 8 mg | 8 mg | --- | 8 mg |
| Borretschöl | 350 mg | 350 mg | --- | 350 mg |
| Leinöl | 50 mg | 50 mg | --- | 50 mg |

### 3) Hilfsstoffe zur galenischen Formulierung:

| | | | | |
|---|---|---|---|---|
| Gelatine, Glycerin, Sorbit, Bienenwachs, Sojalecithin, Triglyceride, Farbstoffe, | q.s | q.s. | --- | q.s. |
| Wasser, Glycerin, Octyldodecanol, Cetylalkohol, Butylenglykol, Triglyceride, Konservierungsstoffe, Aromen | --- | --- | q.s. | --- |

### 4) Vergleichsversuche : Anwendung Haut

Die Zubereitungen Kapsel I, Kapsel IIA + Creme IIB wurden gegen Kapsel III (Vergleich) an freiwilligen Probandinnen (N=6, Alter: 32 - 48 Jahre) in einer Dosierung von 2 Weichgelatine - Kapseln bzw. zweimal 2 g Creme (Typ W/O- Creme) pro Tag über einen Zeitraum von 75 Tagen mittels FOITS (=Fast Optical in-vivo Topometry of Human Skin: M. Rohr, et al., Skin surface claim support by FOITS, SÖFW, Journal, 2-18, 2000; ) sowie mittels ICLS (=lnduzierte Chemilumineszenz: S. Benard, M. Rohr, et al.: Biophotonics -a new efficient testing of UV protection of skin and hair monitored by ICL-Skin and ICL-Hair, SÖFW- Journal 128, 40-45,2002) ermittelt. Dabei zeigte sich eine signifikante Verbesserung der Faltenstruktur zwischen 52 und 86 Prozent im FOITS- Test, während mittels ICLS eine Verbesserung des Hautschutzes vor UV Strahlung von bis zu 73 Prozent zugunsten der erfindungsgemäßen Rezepturen festgestellt werden konnte.

Mit Hilfe von FOITS ermittelt wurde die durchschnittliche Zunahme der mit bloßem Auge nicht sichtbaren Mikrostruktur (<50 µm), die Abnahme der Feinstruktur (50-100 µm) sowie der Grobstruktur (>100 µm) wie aus der Zusammenstellung gemäß Tabelle 5 folgt:

**Tabelle 5**

| Zubereitung / Meßparameter | Zunahme der Mikrostruktur (MS,%) | Abnahme der Feinstruktur (FS%) | Abnahme der Grob struktur (GS%) |
|---|---|---|---|
| Kapsel I | + 6 % (MS-Anteil 64%) | - 4,5% (FS-Anteil 26%) | -24 % (GS-Anteil 10%) |
| Kapsel IIA + Creme IIB | + 7 %(MS-Anteil 72%) | - 5,8 %(FS-Anteil 21%) | -26 %(GS-Anteil: 7%) |
| Kapsel III (Vergleich) | +4 % (MS-Anteil: 42%) | -3,8 % (FS-Anteil: 32%) | -14 %(GS-Anteil: 26%) |
| Max. Verbesserung geg. Vergleich | 75% | 52% | 86% |

Mittels ICLS wurden die Abnahmen der induzierten (provozierten) Photonenemission als integrale Differenzen in Prozent des Ausgangswertes ermittelt und die Ergebnisse für die drei verschiedenen Präparationen verglichen:
Für die erfindungsgemäße Kapsel I ergab sich eine Abnahme von 23 % für die erfindungsgemäße Kombination Kapsel IIA plus Creme IIB ergab sich eine Abnahme von 26 % und für die dem Stand der Technik entsprechende Standardkapsel III eine Abnahme um 15 %. Dies entspricht einer maximalen Verbesserung des Hautschutzes von 73 % für die erfindungsgemäßen Präparationen.

Diese Wirksamkeit ergibt sich auch aus Abbildungen 1 (Kontrolle, unbehandelt) und Abbildung 2 (Kapsel II + AntiAge Creme), die im Auflicht Mikroskop in ca. 100-facher Vergrößerung nach 52-tägiger Anwendung die Verbesserung von Textur und Erscheinungsbild der Haut zeigen.

## Patentansprüche

1. Zubereitungen zur Stabilisierung und zum Aufbau der Textur lebender Gewebe, umfassend eine Kombination aus:
A) einem oder mehreren Inhibitoren des Gewebematrixabbaus durch hydrolytische Enzyme Elastase, Collagenase, Hyaluronidase, ausgewählt aus aus Kurkuma bzw. Kurkuma- Extrakt, Weihrauchextrakt, Persimone, Teufelskrallenwurzel- Extrakt, Olivenöl, Schafgarbe, Rhabarber, Braunalgen / - extrakt oder Grüntee oder Mischungen hiervon;
B) Stimulatoren des Proteoglykan- Matrix-Aufbaus, ausgewählt aus säurehaltigen/säureartigen Substanzen, ausgewählt aus Blaubeeren-/Heidelbeeren-/Preiselbeerenextrakt; Hibiscus-Extrakt; Morinda citrifolia (Nonifrucht); Granatapfelschalenextrakt; Paprikapulver;
und einem oder mehreren
C) gefäßaktiven Substanzen, ausgewählt aus Calendula-Blütenextrakt, Tagetesblütenextrakt; Weiße- Lilien- Extrakt; Tomatenextrakt; Niacin;
und
weiterhin D) einen oder mehrere Aktivatoren der Nervenfunktion (neurotrope Substanzen), ausgewählt aus B- Vitaminen, ausgewählt aus B1, B2, B5, B6, B12, Getreideauszügen wie Buchweizenextrakt aufweist,
wobei die Wirkstoffe A, B, C und D jeweils sowohl in topischer Verabreichungsform als auch in systemischer Verabreichungsform vorliegen, wobei die Zubereitungen in Form galenischer Mittel zur systemischen Verabreichung in Form von Dragees, Kapseln oder Tabletten und zur topischen Verabreichung in Form von Emulsionen/Lotionen, Cremes, Salben, Gelen, Fluids, Sprays vorliegen.

2. Zubereitungen nach Anspruch 1, **dadurch gekennzeichnet, dass** die Kombination als Substanz(en) A einen oder mehrere Elastase- Inhibitoren aufweist.

3. Zubereitungen nach Anspruch 1 oder 2, **dadurch gekennzeichnet, dass** als Komponente A Kurkuma- Wurzel-Extrakt enthalten ist.

4. Zubereitungen gemäß einem der Ansprüche 1 bis 3, **dadurch gekennzeichnet, dass** als Komponente B) Hibiscus - Extrakt, Heidelbeeren-, Preiselbeerenextrakt, Paprikapulver oder Mischungen hiervon ausgewählt ist.

5. Zubereitungen gemäß einem der Ansprüche 1 bis 4, **dadurch gekennzeichnet, dass** als Komponente C) Calendula-Blüten-Extrakt, Tagetesblütenextrakt, Tomatenextrakt, Niacin oder Mischungen hiervon vorhanden sind.

6. Zubereitungen gemäß einem der Ansprüche 1 bis 5, **dadurch gekennzeichnet, dass** als Komponente D) Vitamin B1, B2, B5, B6, B12 oder Mischungen hiervon enthalten sind.

7. Zubereitungen gemäß einem der Ansprüche 1-6 zur Verwendung zur Stabilisierung der Textur bzw. zum Texturaufbau und der Texturneustrukturierung lebender Gewebe von Menschen, insbesondere von Haut und ihrer Anhangsgebilde wie Haarfollikel und Nagelbett oder Bindegewebe, mit traumatisch bedingter oder krankheitsbedingter verstärkter Abbaufunktion.

8. Kosmetische, nicht therapeutische Verwendung von Zubereitungen gemäß einem der Ansprüche 1- 6 zur Stabilisierung der Textur bzw. zum Texturaufbau und der Texturneustrukturierung von Haut.

9. Verwendung gemäß Anspruch 8 zur Verbesserung der Textur und des Erscheinungsbildes von Haut und ihrer Anhangsgebilde Haarfollikel und Nagelbett oder Bindegewebe mit altersbedingter verstärkter Abbaufunktion.

## Claims

1. Preparations for stabilising and forming the texture of living tissue comprising a combination of:
A) one or more inhibitors of tissue matrix breakdown by hydrolytic enzymes elastase, collagenase or hyaluronidase, selected from turmeric or turmeric extract, frankincense extract, persimmon, Devil's Claw root extract, olive oil, milfoil, rhubarb, brown seaweed extract or green tea or mixtures thereof;
B) stimulators of proteoglycan matrix formation, selected from acid-containing/acid-type substances, selected from bilberry/blueberry/ cranberry extract; hibiscus extract; Morinda citrifolia (noni fruit); pomegranate peel extract; paprika;
and one or more
C) vasoactive substances selected from calendula flower extract, tagetes flower extract; white lily extract; tomato extract; niacin;
and
furthermore D) one or more nerve function activators (neurotropic substances), selected from B vitamins, selected from B1, B2, B5, B6, B12, and cereal extracts such as buckwheat extract,
wherein the active substances A), B), C) and D) are in each case present in both a topical administration form and a systemic administration form, wherein the preparations assume the form of pharmaceutical agents for systemic administration in the form of sugar-coated tablets, capsules or tablets and for topical administration in the form of emulsions/lotions, creams, ointments, gels, fluids or sprays.

2. Preparations according to claim 1, **characterised in that** the combination includes one or more elastase inhibitors as substance(s) A).

3. Preparations according to claim 1 or 2, **characterised in that** turmeric root extract is present as component A).

4. Preparations according to one of claims 1 to 3, **characterised in that** hibiscus extract, blueberry or cranberry extract, paprika or mixtures thereof is selected as component B).

5. Preparations according to one of claims 1 to 4, **characterised in that** calendula flower extract, tagetes flower extract, tomato extract, niacin or mixtures thereof are present as component C).

6. Preparations according to one of claims 1 to 5, **characterised in that** vitamin B1, B2, B5, B6, B12 or mixtures thereof are present as component D).

7. Preparations according to one of claims 1-6 for use for stabilising the texture or for texture forming and texture engineering of human living tissue, in particular of skin and its appendages such as hair follicles and nail bed or connective tissue, undergoing increased breakdown as a result of injury or illness.

8. Cosmetic, non-therapeutic use of preparations according to one of claims 1-6 for texture stabilisation or texture forming and texture engineering of skin.

9. Use according to claim 8 for improving the texture and appearance of skin and its appendages such as hair follicles and nail bed or connective tissue undergoing increased age-related breakdown.

## Revendications

1. Préparations pour la stabilisation et l'élaboration de la texture de tissus vivants, comprenant une combinaison
A) d'un ou de plusieurs inhibiteurs de la dégradation de la matrice tissulaire par les enzymes hydrolytiques telles que l'élastase, la collagénase, l'hyaluronidase, choisis parmi le curcuma ou un extrait de curcuma, un extrait d'encens, le plaqueminier, un extrait de racine de griffe du diable, l'huile d'olive, le millefeuille, la rhubarbe, un extrait d'algues brunes ou le thé vert ou leurs mélanges;
B) de stimulateurs de l'élaboration de la matrice de protéoglycane, choisis parmi les substances contenant un acide/de type acide, choisies parmi un extrait de myrtille/airelle/canneberge, un extrait d'hibiscus, le morinda citrifolia (nono), un extrait de peau de grenade, la poudre de paprika;
et
C) d'une ou de plusieurs substances vasoactives, choisies parmi un extrait de fleurs de calendula, un extrait de fleurs de tagète, un extrait de lilas blanc, un extrait de tomates, la niacine;
et
en outre D) d'un ou de plusieurs activateurs de la fonction nerveuse (substances neurotropes), choisis parmi les vitamines B, choisies parmi la vitamine B1, B2, B5, B6, B12, les extraits de céréales tel que l'extrait de sarrasin,
les principes actifs A, B, C et D se trouvant à chaque fois tant sous une forme d'administration par voie topique que sous une forme d'administration par voie systémique, les préparations sous forme d'agents galéniques destinés à l'administration par voie systémique se trouvant sous forme de dragées, de capsules ou de comprimés et, pour l'administration par voie topique, sous forme d'émulsions/lotions, de crèmes, de pommades, de gels, de fluides et de sprays.

2. Préparations selon la revendication 1, **caractérisées en ce que** la combinaison présente un ou plusieurs inhibiteurs d'élastase en tant que substance(s) A).

3. Préparations selon la revendication 1 ou 2, **caractérisées en ce qu'**un extrait de racine de curcuma est contenu en tant que composant A).

4. Préparations selon l'une quelconque des revendications 1 à 3, **caractérisées en ce qu'**un extrait d'hibiscus, un extrait d'airelle, un extrait de canneberge, la poudre de paprika ou leurs mélanges sont choisis comme composant B).

5. Préparations selon l'une quelconque des revendications 1 à 4, **caractérisées en ce qu'**un extrait de fleurs de calendula, un extrait de fleurs de tagète, un extrait de tomates, de la niacine ou leurs mélanges sont présents en tant que composant C).

6. Préparations selon l'une quelconque des revendications 1 à 5, **caractérisées en ce que** les vitamines B1, B2, B5, B6, B12 ou leurs mélanges sont contenus en tant que composant D).

7. Préparations selon l'une quelconque des revendications 1 à 6 pour une utilisation pour la stabilisation de la texture ou pour l'élaboration de la texture et la restructuration de la texture de tissus vivants d'êtres humains, en particulier de la peau et de ses phanères tels que les follicules capillaires et le lit de l'ongle ou le tissu conjonctif, présentant une fonction de dégradation provoquée par un traumatisme ou renforcée par une maladie.

8. Utilisation cosmétique, non thérapeutique, de préparations selon l'une quelconque des revendications 1 à 6 pour la stabilisation de la texture ou pour l'élaboration de la texture et la restructuration de la texture de la peau.

9. Utilisation selon la revendication 8 pour l'amélioration de la texture et de l'aspect de la peau et de ses phanères, tels que les follicules capillaires et le lit de l'ongle ou le tissu conjonctif présentant une fonction renforcée de la dégradation provoquée par la vieillesse.
